# EUROPEAN PATENT APPLICATION

(11) **EP 1 437 118 A1**
(43) Date of publication of application: **14.07.2004**
(21) Application number: 02770221.6
(22) Date of filing: 27.09.2002
(51) Int. Cl.: A61K 7/00, A61K 47/32, A61K 7/48, A61K 9/08

(54) **BASE FOR EXTERNAL PREPARATIONS FOR THE SKIN AND COSMETICS CONTAINING THE SAME**

(30) Priority: 28.09.2001 JP 2001304030
(71) Applicant: Shiseido Co., Ltd., Tokyo 104-8010 (JP); NOF CORPORATION, Tokyo 150-6019 (JP)
(72) Inventor: OHMORI, Takashi, c/o Shiseido Research Center, Yokohama-shi, Kanagawa 224-8558 (JP); KAKOKI, Hiroyuki, c/o Shiseido Research Center, Yokohama-shi, Kanagawa 224-8558 (JP); MIYAHARA, Reiji, c/o Shiseido Research Center, Yokohama-shi, Kanagawa 224-8558 (JP); NANBA, Tomiyuki, c/o Shiseido Research Center, Yokohama-shi, Kanagawa 224-8558 (JP); NAKANE, Toshihiko, c/o Shiseido Research Center, Yokohama-shi, Kanagawa 224-8558 (JP); MARUYAMA, Kei-ichi, Kawasaki-shi, Kanagawa 212-0055 (JP); SAKODA, Takeshi, Kawasaki-shi, Kanagawa 210-0804 (JP); YASUKOHCHI, Tohru, Yokohama-shi, Kanagawa 224-0033 (JP); TEZUKA, Yoji, Kawasaki-shi, Kanagawa 210-0804 (JP)
(74) Representative: Gudat, Axel, Dr.
(86) International application number: PCT/JP2002/010017
(87) International publication number: WO 2003/028672

(57) **Abstract**

A base for a skin external composition comprises, as a main ingredient, an alkylene oxide derivative represented by the following formula (I):

R¹O-[(AO)ₘ(EO)ₙ]-R² (I)

wherein AO is an oxyalkylene group having a carbon number of 3 to 4; EO is an oxyethylene group; and R¹ and R² may be the same or different, and are a hydrocarbon group having a carbon number of 1 to 4, or a hydrogen atom. The base is a water-soluble base that can exert both of the smooth feeling and the moist feeling.

## Description

### RELATED APPLICATIONS

This application claims priority to the Japanese Patent Application No. 2001-304030 filed on September 28, 2001, which is incorporated herein by reference.

### FIELD OF THE INVENTION

The present invention relates to a base for a skin external composition and cosmetics and, in particular, to improvement of the feeling thereof.

### BACKGOUND OF THE INVENTION

A base used in cosmetics can be roughly classified into an aqueous base and an oily base. The oily base, a representative of which includes a triglyceride and a silicone oil, is aimed mainly at imparting the sticky-free feeling and the smooth feeling to a skin, while the aqueous base, a representative of which includes glycerin, propylene glycol and polyethylene glycol, is aimed mainly at imparting the moist feeling to a skin.

However, these oily base and aqueous base have the low compatibility with each other. Therefore, when these two bases are blended in order to impart both of the smooth feeling and the moist feeling, a large amount of an emulsifier is required. However, a large amount of an emulsifier promotes the sticky feeling, loses the moist feeling, and becomes a negative factor for appearance and stability of the composition. For this reason, a base sufficiently satisfying both of the smooth feeling and the moist feeling has never been obtained.

### SUMMARY OF THE INVENTION

The present invention was done in view of the problems in the prior art, and an object of the present invention is to provide a base for a skin external composition excellent in the feeing of use.

In order to attain the aforementioned object, the present inventors extensively studied and, as a result, found that a specified alkylene oxide derivative has the excellent usability as a base for a skin external composition, which resulted in completion of the present invention.

That is, the base for a skin external composition of the present invention comprises, as a main ingredient, an alkylene oxide derivative represented by the following formula (I):

R¹O-[(AO)ₘ(EO)ₙ]-R² (I)

wherein AO is an oxyalkylene group having a carbon number of 3 to 4 and EO is an oxyethylene group, said oxyalkylene group having a carbon number of 3 to 4 and said oxyethylene group may be in a block addition or a random addition;
m and n are average addition mole numbers of the oxyalkylene group and the oxyethylene group, respectively, within a range of 1≦m≦70 and 1≦n≦70, wherein a ratio of the oxyethylene group to a sum of the oxyalkylene group having a carbon number of 3 to 4 and the oxyethylene group is 20 to 80% by weight; and
R¹ and R² may be the same or different, and are a hydrocarbon group having a carbon number of 1 to 4 or a hydrogen atom, wherein a ratio of the number of hydrogen atoms to the number of hydrocarbon groups of R¹ and R² is 0.15 or smaller.

In said base, it is preferable that the oxyalkylene group and the oxyethylene group are in a random addition.

The cosmetic composition of the present invention is characterized in that said base for a skin external composition is incorporated at 0.01 to 70% by weight.

### BEST MODE FOR CARRYING OUT THE INVENTION

In the alkylene oxide derivative represented by the formula (I), AO is an oxyalkylene group having a carbon number of 3 to 4. Examples thereof include oxypropylene, oxybutylene, oxyisobutylene, oxytrimethylene and oxytetramethylene groups, preferably oxypropylene or oxybutylene group.

In the derivative, m is an average addition mole number of the oxyalkylene group having a carbon number of 3 to 4, within 1≦m≦70, preferably 2≦m≦30, more preferably 2 ≦m≦20. n is an average addition mole number of an oxyethylene group, within 1≦n≦ 70, preferably 2≦m≦30, more preferably 2≦n≦20. When the number of the oxyalkylene group having a carbon number of 3 to 4 or the oxyethylene group is 0, the moist feeling is deteriorated. On the other hand, when the number exceeds 70, the feeling becomes sticky and can not becomes smooth sufficiently.

In addition, a ratio of the oxyethylene group to a sum of the oxyalkylene group having a carbon number of 3 to 4 and the oxyethylene group is preferably 20 to 80% by weight, more preferably 30 to 70% by weight. When the ratio of the oxyethylene group is smaller than 20% by weight, the moist feeling tends to be inferior and, when the ratio exceeds 80% by weight, the smooth feeling tends to be inferior.

The addition order of the ethylene oxide and the alkylene oxide having a carbon number of 3 to 4 is not particularly limited. The oxyethylene group and the oxyalkylene group having a carbon number of 3 to 4 may be in a block addition or a random addition. The block addition includes not only 2 step-wise block but also 3 step-wise block. Preferable is a random addition.

R¹ and R² are a hydrocarbon group having a carbon number of 1 to 4 or a hydrogen atom. Examples of the hydrocarbon group include methyl, ethyl, n-propyl, isopropyl, n-butyl, sec-butyl, and tert-butyl groups. Preferable are methyl or ethyl group, and more preferable is methyl group. In the case of a hydrocarbon group having a carbon number of 5 or more, the hydrophilicity is reduced, and the moist feeling is deteriorated. R¹ and R² may be the same or different.

As R¹ and R², only one kind of a variable may be present, or both of a hydrocarbon group having a carbon number of 1 to 4 and a hydrogen atom may be present. Alternatively, different hydrocarbon groups having a carbon number of 1 to 4 may be present. However, in hydrocarbon groups of R¹ and R², a ratio of the hydrocarbon groups and the hydrogen atoms to be present is such that Y/X is 0.15 or smaller, preferably 0.06 or smaller, said Y/X is a ratio of the number of hydrogen atoms (Y) to the number of hydrocarbon groups (X). When the ratio of Y/X exceeds 0.15, the feeling becomes sticky.

The base for a skin external composition of the present invention can be prepared by the known method. For example, a compound having a hydroxy group is addition-polymerized with both of ethylene oxide and alkylene oxide having a carbon number of 3 to 4, and then subjected to an ether reaction with an alkyl halide in the presence of an alkali catalyst, thereby obtaining the base.

The skin external composition of the present invention is an external composition included "cosmetics" and "quasi-drug" defined in Japanese Pharmaceutical Affairs Law. A form of such cosmetics containing the base for a skin external composition is not particularly limited, and may be any of an aqueous cosmetic, a water-in-oil or oil-in-water emulsion cosmetic, and an oily cosmetic. It may be various forms such as shampoo, rinse, body soap, face wash, skin lotion, milky lotion, cream, hairdressing, rouge, and hair growth preparation.

An amount of the base for a skin external composition to be incorporated into the cosmetic of the present invention is not particularly limited, but the base is incorporated at usually around 0.01 to 70% by weight, preferably 0.5 to 40% by weight, further preferably 0.5 to 20% by weight.

### EXAMPLES

Examples of the present invention will be explained below.

First, the present inventors assessed various bases for a skin external composition about the water-solubility, the sticky feeling and the moist feeling based on the following criteria:

### (Water-solubility test)

An aqueous solution containing 10% by weight of the compound was prepared, and its state was observed. Completely dissolved state is most preferable, but the effect of the present invention can be exerted even in the emulsified state. In the insoluble state, the effect can not be obtained. For this reason, completely dissolved or emulsified or more soluble was regarded to be effective.
○: Completely dissolved
Δ: Emulsified
×: Insoluble

### (Organoleptic assessment)

An aqueous solution containing 10% by weight of the compound was prepared, and the sticky-free feeling (smooth feeling) and the moist feeling were assessed using 10 professional panelists. As an assessment method, an upper arm part was washed, and then a sample was applied thereon. From immediately after the application to 30 minutes after the application, the sticky-free feeling and the moist feeling were evaluated according to the five-grade system. When a total score obtained thereby was thirty-five or higher, it was determined to be favorable.

### Sticky-free feeling (smooth feeling)

5: Very spreadable and, when a skin was rubbed, not frictional and very soft touch
4: Spreadable and, when a skin was rubbed, not frictional and soft touch
3: Slightly less spreadable and, when a skin was rubbed, bit frictional touch
2: Less spreadable and, when a skin was rubbed, frictional and heavy touch
1: Much less spreadable and, when a skin was rubbed, frictional and very heavy touch

### Moist feeling

5: Not dry at all, and sufficiently moist touch
4: Not dry, and moist touch
3: Slightly dry, and slightly less moist touch
2: Rough, and less moist touch
1: Very dry, and much less wet

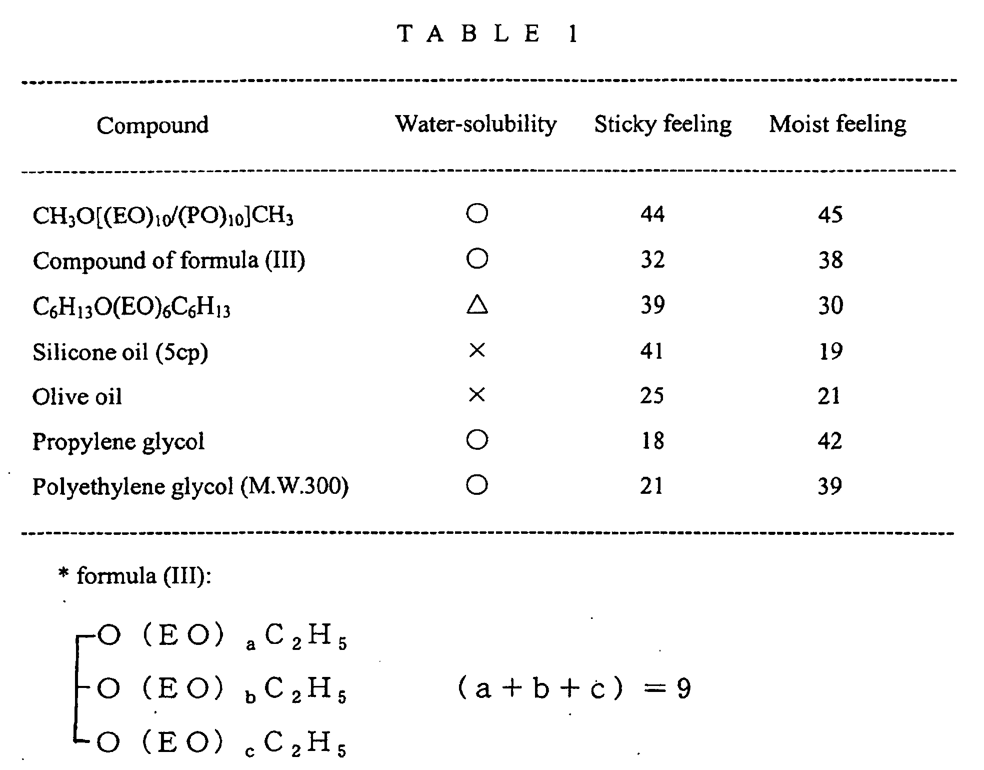

As a result, it was revealed that the EO/PO derivative having hydrocarbon groups attached to both ends thereof is excellent in the sticky feeling and the moist feeling as compared with a general base for a skin external composition such as silicone oil and olive oil. Then, the present inventors further studied the compound represented by formula I. Respective compounds shown below were prepared according to Synthesis Example 1 or 2.

### Synthesis Example 1 Synthesis example of random polymer

### Polyoxyethylene (10mol) polyoxypropylene (10mol) dimethyl ether (Compound 2) CH₃O[(EO)₁₀/(PO)₁₀]CH₃

In the following Examples, EO represents an oxyethylene group, PO represents an oxypropylene group, and [(EO)/(PO)] represents a random bond.

76g of propylene glycol and 3.1g of potassium hydroxide as a catalyst were placed into an autoclave. After the air in the autoclave was replaced with dry nitrogen, the catalyst was completely dissolved at 140 °C while stirring. Then, a mixture of 440g of ethylene oxide and 522g of propylene oxide was added dropwise thereto by a dropping device, and the mixture was stirred for 2 hours. Then, 224g of potassium hydroxide was placed thereinto. After the system was replaced with dry nitrogen, 188g of methyl chloride was injected therein at a temperature of 80 to 130 °C and a reaction was performed for 5 hours. Thereafter, the reaction composition was taken out from the autoclave, neutralized with hydrochloric acid to pH 6 to 7, and then treated at 100 °C for 1 hour under reduced pressure of 0.095MPa (50mmHg) to remove the contained water. After the treatment, filtering was performed to remove the produced salt, to give a base of Compound 2.

Before the reaction with methyl chloride, sampling was performed. The sample purified had a hydroxyl value of 107, Compound 2 had a hydroxyl value of 0.4, and a ratio of the number of hydrogen atoms to the number of terminal methyl groups was 0.004. Therefore, the hydrogen atom was almost completely converted to methyl group.

### Synthesis Example 2 Synthesis of block polymer

### Polyoxyethylene 10mol) polyoxypropylene (10mol) dimethyl ether (Compound 9) CH₃O(EO)₅(PO)₁₀(EO)₅CH₃

76g of propylene glycol and 3.1 g of potassium hydroxide as a catalyst were placed into an autoclave. After the air in an autoclave was replaced with dry nitrogen, and the catalyst was completely dissolved at 140 °C while stirring. Then, 522g of propylene oxide was added dropwise thereto by a dropping device, and the mixture was stirred for 2 hours. Subsequently, 440g of ethylene oxide was added dropwise thereto by a dropping device, and the mixture was stirred for 2 hours. Then, 224g of potassium hydroxide was placed thereinto. After the system was replaced with dry nitrogen, 188g of methyl chloride was injected therein at 80 to 130 °C and a reaction was performed for 5 hours. Thereafter, the reaction composition was taken out from the autoclave, neutralized with hydrochloric acid to pH 6 to 7, and then treated at 100 °C for 1 hour under reduced pressure of 0.095MPa (50mmHg) to remove the contained water. After the treatment, filtering was performed to remove the produced salt, to give a base of Compound 9.

Before the reaction with methyl chloride, sampling was performed. The sample purified had a hydroxyl value of 110, Compound 9 had a hydroxyl value of 0.3, and a ratio of the number of hydrogen atoms to the number of terminal methyl groups was 0.003. Therefore, the hydrogen atom was almost completely converted to methyl group.

According to the forgoing respective Preparation Examples, various alkylene oxide derivatives were prepared and assessed as a base for a skin external composition.

### (Determination of R¹, R²)

First, the present inventors assessed R¹ and R² as well as the suitability as a base for a skin external composition. The results are shown in the following Table 2. In any compounds, as EO and PO parts,

[(EO)₁₀/(PO)₁₀]

is used.

A ratio of unsubstitution is expressed as Y/X, which is a ratio of the number of hydrogen atoms (Y) to the number of hydrocarbon groups (X).

**TABLE 2**

| Compound | R¹ | R² | Y/X | Water-solubility | Sticky feeling | Moist feeling |
|---|---|---|---|---|---|---|
| 1 | H | H | - | ○ | 31 | 40 |
| 2 | CH₃ | CH₃ | 0.004 | ○ | 44 | 45 |
| 3 | C₁₂H₂₅ | CH₃ | 0.006 | Δ | 36 | 33 |

As apparent from Table 2, the excellent water-solubility and feeling of use were obtained when a carbon number of both of R¹ and R² is 1 to 4 (Compound 2).

To the contrary, when R¹ and R² are a hydrogen atom (Compound 1), the sticky feeling is strong. When R¹ is C12 (Compound 3), the water-solubility is deteriorated, and the sticky feeling and the moist feeling were both not preferable.

Since all of R¹ and R² are not always substituted with a hydrocarbon group upon actual preparation, an acceptable existence ratio of the unsubstituted (H) compound was studied. In the following Table 3, 1:2 = 5:95 means that Compound 1 and Compound 2 were mixed at a ratio of 5:95 to adjust to a predetermined Y/X.

**TABLE 3**

| Compound | R¹ | R² | Y/X | Water-solubility | Sticky feeling | Moist feeling |
|---|---|---|---|---|---|---|
| 1 | H | H | - | ○ | 31 | 40 |
| 2 | CH₃ | CH₃ | 0.004 | ○ | 44 | 45 |
| 1 :2= 5:95 | | | 0.053 | ○ | 39 | 42 |
| 1:2=20:80 | | | 0.202 | ○ | 33 | 41 |

As apparent from the above Table 3, even when R¹ and R² are partially unreacted, if an amount of unreacted R¹ or R² is small (Y/X=0.053), there is no great influence. However, when Y/X becomes 0.202, the sticky feeling is clearly produced. The present inventors further studied in detail and, as a result, it was revealed that Y/X is necessary to be 0.15 or smaller.

### (Oxyalkylene and oxyethylene groups)

Then, the present inventors studied the presence of the oxyalkylene group and the oxyethylene group as well as the suitability as a base for a skin external composition.

The results are shown in the following Table 4. In all of compounds, R¹ and R² are each CH₃. In all of compounds, the part of [(EO)/(PO)] is in a random addition.

**TABLE 4**

| Compound | EO | PO | Y/X | Water-solubility | Sticky feeling | Moist feeling |
|---|---|---|---|---|---|---|
| 4 | 20 | 0 | 0.010 | ○ | 30 | 39 |
| 5 | 15 | 5 | 0.010 | ○ | 41 | 35 |
| 2 | 10 | 10 | 0.004 | ○ | 44 | 45 |
| 6 | 6 | 14 | 0.008 | ○ | 36 | 35 |
| 7 | 0 | 20 | 0.019 | × | 37 | 23 |
| 8 | 25 | 25 | 0.004 | ○ | 36 | 40 |

As apparent from Table 4, the presence of both of the oxyalkylene group and the oxyethylene group is indispensable for the base of the present invention. By further detailed study by the present inventors, it was revealed that a suitable ratio of the oxyethylene group to a sum of the oxyalkylene group and the oxyethylene group is 20 to 80 % by weight.

Further, the present inventors prepared a block polymer and a random polymer at the same alkylene group number and oxyethylene group number, and they were compared.

**TABLE 5**

| Compound | EO | PO | Y/X | Water-solubility | Sticky feeling | Moist feeling |
|---|---|---|---|---|---|---|
| 9 | CH₃O(EO)₅(PO)₁₀(EO)₅CH₃ | | 0.003 | ○ | 39 | 40 |
| 2 | CH₃O[(EO)₁₀/(PO)₁₀]CH₃ | | 0.004 | ○ | 44 | 45 |

As apparent from the above Table 5, even a block polymer or a random polymer can extert the effects of the present invention and, in particular, in the case of a random polymer, the excellent feeling of use can be obtained.

### Incorporation in cosmetics

Then, the present inventors actually incorporated various compounds into cosmetics, and assessed them.

The compound, propylene glycol or polyethylene glycol (molecular weight 300) shown in Table 6(hereinafter, test compound) was incorporated into an aqueous phase part. An oily phase part out of the base consisted of the following composition was warmed to 60 °C to be uniformly dissolved. Then, the aqueous phase part was added to the oil phase part at the same temperature while stirring, to prepare a cream, which was assessed as described above.

| | | |
|---|---|---|
| Oil phase part | Cetanol | 2.0 wt% |
| | Beeswax | 6.0 wt% |
| | Petrolatum | 5.0 wt% |
| | Squalane | 34.0 wt% |
| | Isopropylmyristate | 5.0 wt% |
| | Glyceryl monostearate | 2.0 wt% |
| | Stearic acid | 0.5 wt% |
| | Polyoxyethylene (18mol) dodecyl ether | 1.5wt% |
| | Perfume | q.s. |
| | Antiseptics | q.s. |
| | Test compounds | 4.0 wt% |
| | Purified water | Balance |

The prepared creams were subjected to the organoleptic assessment.

**TABLE 6**

| Compound | EO weight ratio | Sticky feeling | Moist feeling |
|---|---|---|---|
| 1 | 43 | 34 | 37 |
| 2 | 43 | 42 | 40 |
| 3 | 43 | 36 | 32 |
| 4 | 100 | 33 | 38 |
| 5 | 69 | 41 | 35 |
| 6 | 24 | 37 | 36 |
| 8 | 43 | 35 | 39 |
| 9 | 43 | 38 | 37 |
| Formula (III) | 100 | 34 | 38 |
| C₆H₁₃O(EO)₆C₆H₁₃ | 100 | 38 | 31 |
| Propylene glycol | - | 32 | 39 |
| Polyethylene glycol (M.W.300) | 100 | 33 | 36 |

As apparent from Table 6, like the results of assessment of a single base aforementioned, each Compounding Example containing the base of the present invention (Compound 2, 5, 6, 8 or 9) satisfies both scores of the sticky-free feeling and the moist feeling, and it can be understood that the base has a function as a base for a skin external composition such as a light feeling. However, other Comparative Examples do not satisfy two properties at the same time.

As explained above, the base for a skin external composition and cosmetics containing the same have both properties of the excellent smooth feeling and moist feeling, and have a favorable touch. Accordingly, the base can exert the most effective performance when incorporated in cosmetics.

## Claims

1. A base for a skin external composition comprises, as a main ingredient, an alkylene oxide derivative represented by the following formula (I):
R¹O-[(AO)ₘ(EO)ₙ]-R² (I)
wherein AO is an oxyalkylene group having a carbon number of 3 to 4 and EO is an oxyethylene group, said oxyalkylene group having a carbon number of 3 to 4 and said oxyethylene group may be in a block addition or a random addition;
m and n are average addition mole numbers of the oxyalkylene group and the oxyethylene group, respectively, within a range of 1≦m≦70 and 1≦n≦70, wherein a ratio of the oxyethylene group to a sum of the oxyalkylene group having a carbon number of 3 to 4 and the oxyethylene group is 20 to 80% by weight; and
R¹ and R² may be the same or different, and are a hydrocarbon group having a carbon number of 1 to 4 or a hydrogen atom, wherein a ratio of the number of hydrogen atoms to the number of hydrocarbon groups of R¹ and R² is 0.15 or smaller.

2. The base for a skin external composition according to the claim 1, wherein the oxyalkylene group and the oxyethylene group are in a random addition.

3. A cosmetic composition wherein the base for a skin external composition according to claim 1 or 2 is incorporated at 0.01 to 70% by weight.
